# EUROPEAN PATENT SPECIFICATION

(11) **EP 0 709 056 B1**
(45) Date of publication and mention of the grant of the patent: **14.01.2004**
(21) Application number: 95202901.5
(22) Date of filing: 26.10.1995
(51) Int. Cl.: A61B 1/12

(54) **Apparatus for cleaning and disinfection of endoscopes**
Vorrichtung zum Reinigen und Sterilisieren von Endoskopen
Dispositif destinés au nettoyage et à la stérilisation d'endoscopes

(30) Priority: 27.10.1994 NL 9401788
(43) Date of publication of application: 01.05.1996
(73) Proprietor: Dyped B.V., 1435 KR Rijsenhout (NL)
(72) Inventor: Meininger, Jules, NL-3927 BJ Renswoude (NL); van Nierop, Adrianus Jacobus, NL-2351 GS Leiderdorp (NL); van Nierop, Jacob, NL-4791 KG Klundert (NL)
(74) Representative: Iemenschot, Johannes Andreas, Ir.

(56) References cited:
- EP-A- 0 271 157
- EP-A- 0 483 059
- EP-A- 0 603 563
- US-A- 2 845 930

## Description

The invention relates to an apparatus for cleaning and disinfection of endoscopes according to the preamble of claim 1 as known from document EP-A-0 483 059.

Such an apparatus is also known from EP-A-0,271,157. It is stated in this publication that pressure sensors can be used to determine whether a specific resistance is encountered in the endoscope channels to be flushed.

The object of the invention is to provide an improved apparatus for cleaning and disinfection of endoscopes, in which in particular the testing of the permeability of the endoscope channels and in particular of the very narrow endoscope channels is improved.

This object is achieved according to the invention by the apparatus according to claim 1.

Preferred embodiments of the apparatus according to the invention are described in the subclaims.

The invention will be explained in greater detail below with reference to the drawings, in which:
Fig. 1 shows a very diagrammatic view in perspective of the apparatus according to the invention;
Fig. 2 shows a rear view on a reduced scale of the apparatus of Fig. 1;
Fig. 3 is the flow chart of a system present in the apparatus according to the invention for cleaning and disinfection of an endoscope;
Fig. 4 is a detail of Fig. 3 showing the diagram of the flow-through unit.
Figs. 5 - 9 are views, partially in section, of a flow-through test unit incorporated in the system of Fig. 3, viewed in the direction of the arrows V-IX.

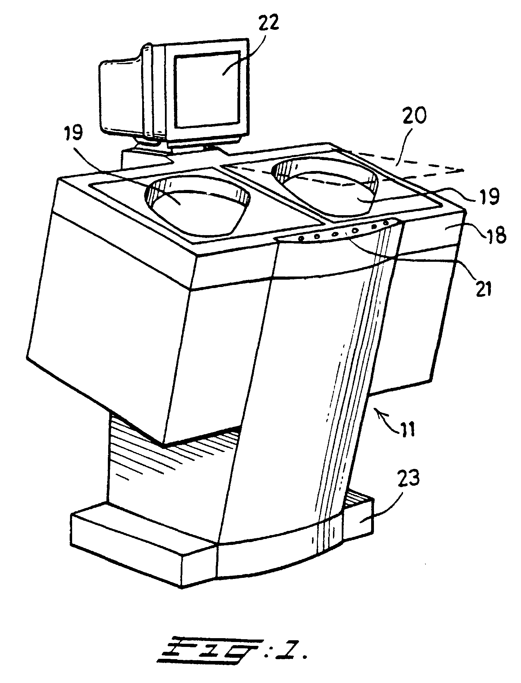

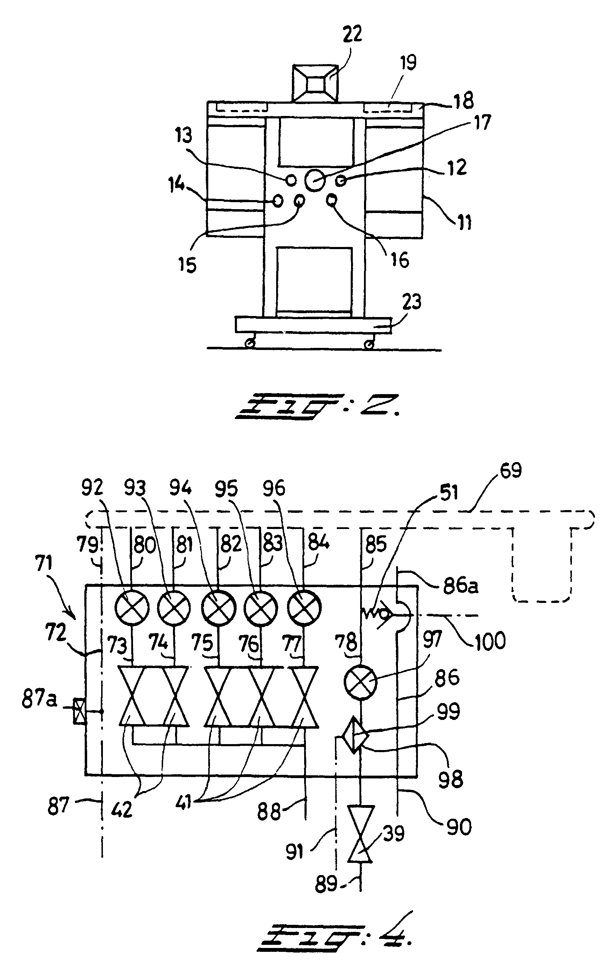

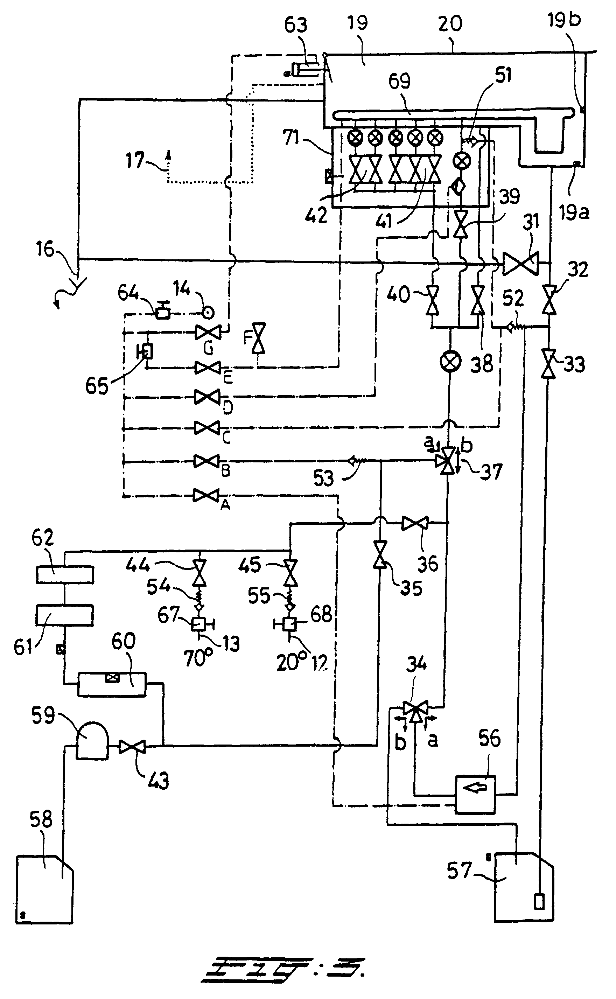

Figs. 1 and 2 show diagrammatically an apparatus for cleaning and disinfection of endoscopes. This apparatus comprises a housing 11, in which two identical systems for cleaning and disinfection of an endoscope, as shown in Fig. 3, are accommodated. The housing is provided with connections 12 - 15 for cold water (20°C), hot water (70°C), compressed air and electricity (220V), an outlet 16 for spent liquids which can be connected to the sewer, and an air outlet 17 which can be connected to an extraction unit.

The housing also has a top piece 18, in which two washbasins 19 are accommodated, each of the two washbasins being able to accommodate an endoscope for cleaning and disinfection. The capacity of each washbasin 19 is approximately 5 litres. Each washbasin 19 is formed in such a way that when said washbasin is filled with liquid an endoscope for cleaning and disinfection is completely immersed in liquid. Each washbasin 19 is provided at the top side with a cover 20. Two endoscopes can be treated simultaneously and independently of each other.

The device is also provided with a control panel 21 and a screen 22.

The housing 11 stands on a base 23 which, as shown in Fig. 2, can be provided with wheels, so that the apparatus is mobile.

Fig. 3 shows the flow chart of one of the systems accommodated in the apparatus according to the invention for cleaning and disinfection of an endoscope. In this chart the liquid pipes are indicated by solid lines, the compressed air lines by dashed-and-dotted lines, and the vent line by a dotted line. Sensors are indicated by a circle or a rectangle containing a cross. Liquid valves 31 - 45 are accommodated in the liquid pipes, and air valves A - G are accommodated in the air lines. The system also contains non-return valves 51 - 55, a pneumatic pump 56, a stock tank 57 for a disinfectant, a stock tank 58 for a cleaning agent (soap), a peristaltic pump 59, a heating element 60, a bacteria filter 61 with a passage of 0.2 µm, a water filter 62 with a passage of 0.8 µm, an air cylinder 63 for operation of the cover 20 of the washbasin 19, and reducing valves 64 - 68. The endoscope for cleaning and disinfection is indicated by 69. The washbasin 19 contains a lower level sensor 19a and an upper level sensor 19b, the lower level sensor 19a detecting that all liquid has flowed out of the washbasin 19, and the upper level sensor 19b detecting that the washbasin 19 is sufficiently filled with liquid.

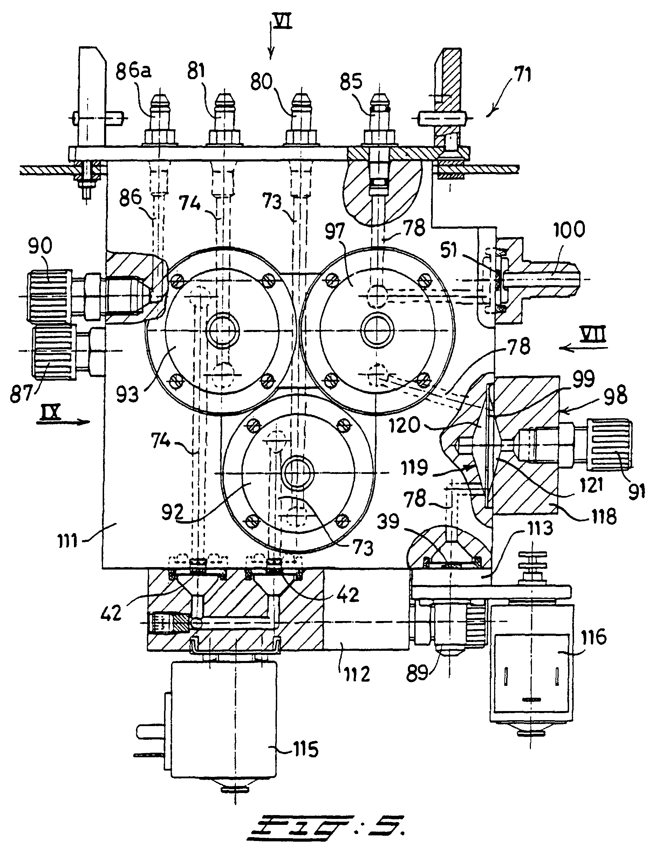

The system shown in Fig. 3 comprises a flow-through test unit 71, indicated in its entirety by 71, and shown separately and on an enlarged scale in Fig. 4. The flow-through test unit 71 is provided with a number of channels 72 - 78 with outlets 79 - 85, which can be connected to an endoscope 69 accommodated in the washbasin 19 and to a channel 86, the outlet 86a of which is connected to the washbasin. The flow-through test unit also has a number of inlets 87 - 90 for the channels 72 -78 and 86, a compressed air connection 91, and a compressed air connection 100. The inlets 87 - 90, the compressed air connection 91 and the compressed air connection 100 are connected to the rest of the system shown in Fig. 3. A valve 39, 41, 42 and a pressure sensor 92 - 97, fitted between the valve 39, 41, 42 and the outlet 80 - 85 are accommodated in each channel 73 - 78 leading to an outlet 80 - 85, which can be connected to an endoscope channel. The pressure sensors are preferably in the form of pressure switches.

A pressure-increasing pump 98 is accommodated in the channel 78, between the valve 39 and the pressure sensor 97. Said pressure-increasing pump 98 is in the form of a pneumatically operated (compressed air connection 91) diaphragm pump with a diaphragm 99.

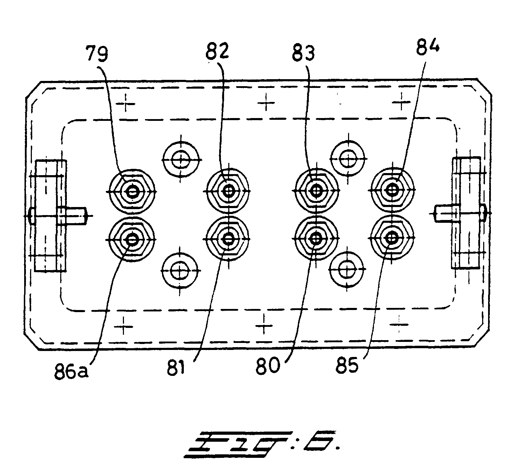

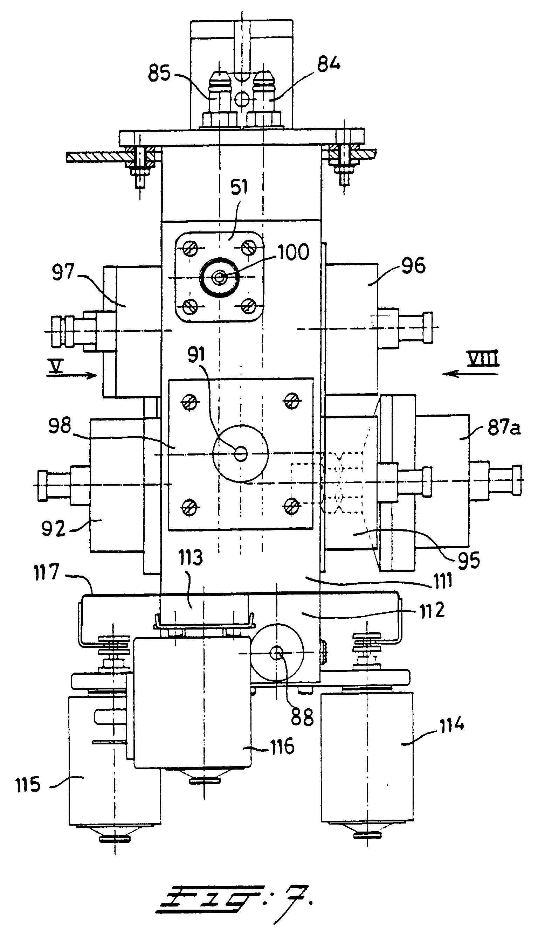

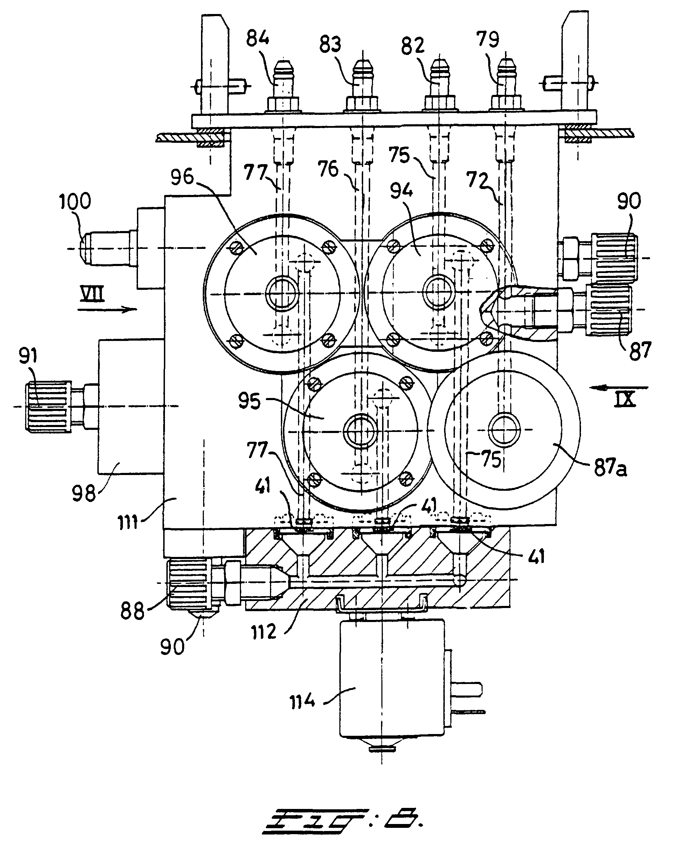

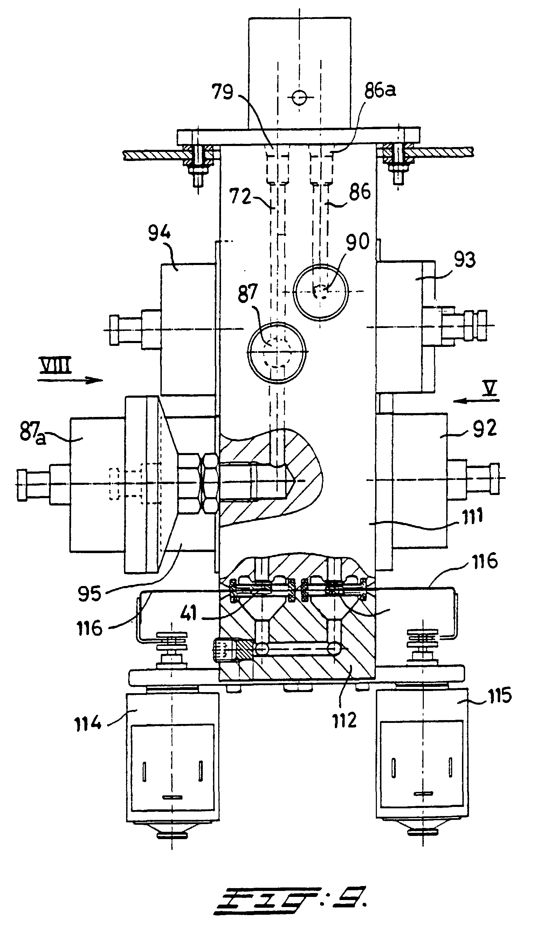

Figures 5 - 9 show a practical embodiment of the flow-through test unit shown diagrammatically in Fig. 4. In Figures 5 - 9 the various parts of the flow-through test unit are indicated by the same reference numbers as in Fig. 4.

The flow-through test unit shown in Figures 5 - 9 consists essentially of a first block 111 and a second block 112 connected thereto. The channels 72 - 78 are disposed in the first block 111. The outlets 79 - 85 and 86a of said channels are disposed at the top side of the block 111. The pressure sensors 92 - 97, in the form of pressure switches, are fitted at the front and rear side of the block 111 (see in particular Fig. 5 and Fig. 8). The non-return valve 51 with compressed air connection 100, the inlets 87 and 90 and the diaphragm pump 98 with compressed air connection 91 are disposed at the sides of the block 111.

The valves 41 (see Fig. 8) and 42 (see Fig. 5) are fitted between the first block 111 and the second block 112, in which latter block the channels leading from the inlet 88 to the valves 41 and 42 are accommodated. The valve 39 (see Fig. 5) is fitted between the first block 111 and a third block 113. The valves 41 and 42 are operated by an electromagnet 114 and 115 respectively. The valve 39 is operated by an electromagnet 116.

The valves 41 and 42 are operated by means of a special leaf spring 117 which is formed in one piece.

The pressure-increasing pump, in the form of a pneumatically operated diaphragm pump 98 (see Fig. 5) comprises a chamber 119 which is formed in the first block 111 and a block 118 fixed thereon, which chamber is divided by the diaphragm 99 into two chambers, a liquid chamber 120 and an air chamber 121. The liquid chamber 120 is in communication with the channel 78. The air chamber 121 is in communication with the compressed air connection 91.

The procedure for cleaning and disinfection of an endoscope containing six channels, namely a very narrow channel, the so-called lift channel, three "small" channels and two "large" channels, will be described below. In the case of the lift channel one must imagine a channel with an internal diameter of 0.5 mm, in which a wire with an external diameter of 0.3 mm is accommodated, so that a gap of 0.1 mm remains. This channel is approximately 1.0 to 1.8 m long. The small channels have an internal diameter of, for example, 0.8 mm, and the large channels have an internal diameter of, for example, 2.8 mm. The length of these channels can be 1.5 to 3.9 m.

The endoscope for cleaning and disinfection is placed in a rack and placed with the rack in a washbasin 19 and connected to the appropriate flow-through test unit 71.

Starting from a neutral position, the following phases/steps are distinguished in each cycle of the cleaning and disinfection of an endoscope:
Leak test
   Pre-flushing phase
Filling with water/soap
Water/soap flushing
Flushing with water
Blow-through
   Disinfection phase
Filling with disinfectant
Disinfection
Flow-through test
Disinfectant return
Blow-through disinfectant
   Rinsing phase
Filling with water
Flushing with water
Flow-through test
Emptying washbasin
Blow-through

In the neutral position all valves are not actuated. The valves 31, 41 and 42 are opened, and the other two-way valves are closed. The three-way valves 34 and 37 are in position "a".

### Leak test

For the leak test the valves G and E are opened. By way of the valve G the pneumatic cylinder 63 is actuated, by means of which the cover 20 of the washbasin 19 is closed. By way of the valve E and the channel 72 in the flow-through test unit 71 air at a pressure of 0.24 bar is conveyed to the inside of the endoscope, i.e. into the space between the casing and the channels. The valve E is then closed. If the pressure in the channel 72, which is measured by the pressure sensor 87a, falls from 0.24 bar to 0.14 bar (or another lower value to be set) in a period of 180 sec., a leak is established in one of the channels and/or the outer casing. A precise determination of the location is not possible. When a leak is established the endoscope must not be disinfected, in order to ensure that damage is prevented. After the leak test the endoscope remains under pressure during the cleaning and disinfection. After the disinfection the endoscope must be vented. This is achieved within 30 sec. by opening the valve F.

### Pre-flushing phase

During the pre-flushing phase the endoscope channels are flushed with water and soap at a pressure of 1 bar. In order to obtain soap in the system, the washbasin 19 is first filled by way of valve 38 at least up to the upper level (upper level meter 19b) with cold water (20°C) containing a soap solution, which is added to the water, for example 100 ml soap per litre of water, by the pump 59 from the stock tank 58 and by way of the valve 43. The endoscope channels are flushed with the same water/soap solution, the water flowing by way of the channels 73 - 78 into the flow-through test unit 71 to the endoscope channels. The flushing is carried out by the pressure of the water mains (max. 2.8 bar) and can be interrupted in order to allow the soap to take effect.

A start is made by filling the lift channel of the endoscope and the liquid chamber 120 of the pressure-increasing pump 98, which will be indicated below as the lift pump, by way of the channel 78 in the flow-through test unit 71. During the filling the pressure rises to the water mains pressure. This is indicated by the pressure sensor 97. The forced flushing of the lift channel of the endoscope by means of the lift pump 98 is then started. For this purpose, the valve 39 is closed, and the valve D is opened, as a result of which compressed air at a pressure of 2.5 - 4 bar, preferably 3.2 bar, passes by way of the compressed air connection 91 into the air chamber 121 of the lift pump 98. The soap solution will consequently be forced out of the liquid chamber 120 and through the lift channel.

During the forced flushing of the lift channel, first the small channels and then the large channels of the endoscope are pre-flushed by opening of the valve 40 and the valves 41 and 42.

At the end of the flushing a flow-through test is carried out. The channels in the endoscope are checked for blockages. The test is divided into three parts: 1) lift channel, 2) large channels (extraction and biopsy channel), 3) small channels (air, water and jet channel). In the case of the lift channel the flow-through test starts when the liquid chamber 120 has been forced empty after about 3 minutes and the pressure measured by the pressure sensor 97 falls. When the pressure has fallen below 1.8 bar (after approx. 2.5 minutes), the lift channel is open. Owing to the great internal flow-through resistance of the lift channel, the pressure will not fall below 1.2 when the channel is open. In the event of a blocked or partially blocked lift channel, the liquid chamber 120 will not be forced empty, and the pressure measured by the pressure sensor 97 will not fall.

The flow-through test on the small and large channels can coincide with the forced flushing and the flow-through test of the lift channel. After the pre-flushing with water and soap, the valves 41 are closed and the flow-through test on the small channels starts. In the meantime the large channels are flushed with water and soap. The flow-through test on these channels starts by closing the valves 42. If a channel is blocked, the pressure is maintained between the valve 41, 42 in question and the blockage, and the pressure measured by the pressure sensor 92 - 96 concerned will not fall. If a channel is not blocked, the pressure will fall. If all pressure sensors (pressure switches) 92 - 97 have dropped off, the system passes on to the next process step. If one of the pressure sensors does not drop off, the system moves to alarm after a certain time, and a blockage is indicated by way of the screen.

In order to remove all soap residues, endoscope channels are flushed with water alone. Again, the first step is to fill the lift channel and the lift pump chamber 120, the pressure sensor 97 checking whether the chamber in question is at pressure. The lift pump 98 is then started. After that, the remaining channels of the endoscope are flushed with water.

After the flushing, the water is blown out of the pipes and channels. The volume in the supply pipe of the lift channel is not forced out through the endoscope, but through the filling line, since this takes less time. For this purpose the valves 39 and 38 are opened, and compressed air is supplied through the connection 100 and the non-return valve 51. The lift channel is blown through after the valve 39 has been closed again. The other channels are blown through with compressed air which is supplied by way of non-return valve 53 and valve 40. The endoscope channels are blown dry in order to prevent mixing of water and disinfectant subsequently fed in.

### Disinfection phase

First of all, the pump 56 pumps disinfectant out of the stock tank 57 into the washbasin 19, by way of the valves 33, 34, 37 and 38. The capacity of the stock tank 57 is approximately 5 litres, as is the capacity of the empty washbasin 19. If the washbasin is filled up to the top level (top level sensor 19b), almost all disinfectant present is utilized, and the washbasin is also prevented from overflowing and disinfectant from being lost. For the disinfection, the disinfectant is circulated through the endoscope channels (including the lift channel) and the washbasin for a set period of time in the manner already described above for the pre-flushing. This circulation is carried out by means of the pump 56. The lift pump 98 is used again for the forced flushing for the lift channel. The disinfectant is then pumped back to the stock tank 57. When the washbasin 19 is empty, which is detected by the lower level sensor 19a, the system is blown through again, in order to remove as much as possible of the disinfectant from the system and return it to the stock tank 57. In the disinfection phase a flow-through test can again be carried out, as in the pre-flushing phase.

### Rinsing phase

First of all, the washbasin 19 is partially filled with a small amount of water, and the channels are briefly flushed. The water with a relatively high concentration of disinfectant is discharged. For the rinsing the washbasin 19 is filled completely with water, and the lift channel, the small and the large channels are flushed. Finally, the entire system is again systematically blown through. A flow-through test can also be carried out in the rinsing phase.

After the rinsing phase the endoscope is vented. At the end of each disinfection cycle the cover 20 of the washbasin 19 remains closed, in order to prevent evaporation.

The endoscope is removed from the washbasin by means of the rack. The disinfected endoscope is stored in the rack until it is to be used again.

The disinfectant used is glutaraldehyde (CIDEX®) or an equivalent agent. This agent can be reused on condition that it is not diluted. After each cleaning phase the pipes are therefore blown through and blown dry.

The procedure for cleaning and disinfection is controlled by an electronic control unit. Said unit can identify an endoscope for cleaning and disinfection and, depending on the type of endoscope and values of certain parameters measured during the cycle, can control the process.

The entire procedure can be followed on the screen 22. Faults during the procedure are indicated directly on the screen. Faults which are vital as regards proper disinfection and as regards the endoscope lead to an immediate interruption of the process and a situation which is safe for the user. The duration of the procedure is indicated on the screen, as a result of which efficient use is possible. All completed disinfection cycles are stored step by step from the flushing phase to the end phase in a memory, the time taken for each step also being saved in the memory. A simple and quick selection system makes it possible to call up any desired cycle over a period of six months and to print it out if necessary.

In the case of cleaning systems of the type described above the microbacterium chelonei constitutes a particular problem. This bacterium is present in tap water and can be destroyed by chemical agents. If present for longer periods in the apparatus, the bacterium can lodge in the biofilm produced in the apparatus, and then becomes encapsulated, and consequently becomes resistant to the chemical agents. Said biofilm is produced after quite a long time through the interaction of water and oxygen in the pipe system. Other forms of life can also lodge in such biofilms, with the result that cross-infections between the various endoscopes to be cleaned can occur. In order to avoid the risk of such a cross-infection, the system of pipes, pumps and valves is periodically heat-cleaned. The heat-cleaning is carried out by flushing the entire system with hot water (85°C - 90°C) for a period of at least 30 minutes. The biofilm is broken down and the bacterium removed in this way.

This cleaning is preferably carried out daily (at night). In addition to or instead of the periodic heat-cleaning, it is conceivable for periodic chemical cleaning to be carried out with an agent which is known to remove the biofilm.

## Claims

1. Apparatus for cleaning and disinfection of endoscopes, comprising at least one washbasin (19) in which an endoscope (69) for cleaning and disinfection can be placed, a housing (11) containing, inter alia, a system of pipes, pumps and valves for the supply of liquids and air to an endoscope placed in a washbasin, means for connecting the channels of an endoscope placed in a washbasin to the system of pipes, pumps and valves, and control means for controlling the pumps and valves, the system of pipes, pumps and valves comprising a flow-through test unit (71) provided with channels (72-78) and having a number of outlets (79-85) connecting to the channels, which outlets can be connected to an endoscope (69) placed in a washbasin (19) and a number of inlets (87-90) connecting to the channels, which inlets are connected to the rest of the system of pipes, pumps and valves, while each channel leading to an outlet (80-85) which can be connected to an endoscope channel contains a valve (39, 41, 42) **characterized in that** each channel leading to an outlet (80-85) which can be connected to an endoscope channel contains a pressure sensor (92-97) fitted between the valve (39, 41, 42) and the outlet (80-85) of the channel.

2. Apparatus according to claim 1, **characterized in that** each pressure sensor (92-97) is in the form of a pressure switch.

3. Apparatus according to claim 3, **characterized in that** a pressure-increasing pump (98) is accommodated in at least one of the channels of the flow-through test unit, between the valve (39) and the pressure sensor (97).

4. Apparatus according to claim 3, **characterized in that** the pressure-increasing pump (98) is in the form of a pneumatically operated diaphragm pump.

5. Apparatus according to one or more of claims 1 - 4, **characterized in that** the apparatus is provided with means for periodically cleaning the system of pipes, pumps and valves.

6. Apparatus according to one or more of claims 1 - 4, **characterized in that** the apparatus is provided with means for identifying an endoscope for cleaning and disinfection, means for controlling the cleaning and disinfection cycle depending on the type of endoscope and values of certain parameters measured during the cycle, and means for storing the course of the cleaning and disinfection cycle in a memory.

## Patentansprüche

1. Vorrichtung zum Reinigen und Sterilisieren von Endoskopen mit wenigstens einem Reinigungsbehälter (19), in welchem ein Endoskop (69) zur Reinigung und Desinfektion platzierbar ist, einem Gehäuse (11), welches, unter anderem, ein System von Rohrleitungen, Pumpen und Ventilen zur Zufuhr von Flüssigkeiten und Luft zu einem in einem Reinigungsbehälter platzierten Endoskop aufweist, Mitteln zur Verbindung der Durchlasskanäle eines in einem Reinigungsbehälter platzierten Endoskops mit dem System von Rohrleitungen, Pumpen und Ventilen und mit Steuereinrichtungen zur Steuerung der Pumpen und Ventile, wobei das System von Rohrleitungen, Pumpen und Ventilen eine Durchströmungs-Prüfeinheit (71) aufweist, welche mit Durchlasskanälen (72-78) versehen ist und welche eine Anzahl von an den Durchlasskanälen angeschlossenen Auslässen (79-85) aufweist, welche an einem in einem Reinigungsbehälter (19) platzierten Endoskop (69) anschließbar sind, und eine Anzahl von an den Durchlasskanälen angeschlossenen Einlässen (87-90) aufweist, welche am restlichen System der Rohrleitung, Pumpen und Ventile angeschlossen sind, und wobei jeder zu einem mit einem Endoskop-Durchlasskanal anschließbaren Auslass (80-85) führende Durchlasskanal, ein Ventil (39, 41, 42) aufweist,
**dadurch gekennzeichnet,**
**dass** jeder zu einem an einen Endoskop-Durchlasskanal anschließbaren Auslass (80-85) führende Durchlasskanal einen Drucksensor (92-97) aufweist, welcher zwischen dem Ventil (39, 41, 42) und dem Auslass (80-85) des Durchlasskanals angeordnet ist.

2. Vorrichtung nach Anspruch 1, **dadurch gekennzeichnet, dass** jeder Drucksensor (92-97) als Druckschalter ausgebildet ist.

3. Vorrichtung nach Anspruch 1, **dadurch gekennzeichnet, dass** in wenigstens einem der Durchlasskanäle der Durchströmungs-Prüfeinheit zwischen dem Ventil (39) und dem Drucksensor (97) eine Druckerhöhungs-Pumpe (98) angeordnet ist.

4. Vorrichtung nach Anspruch 3, **dadurch gekennzeichnet, dass** die Druckerhöhungs-Pumpe (98) als pneumatisch betätigte Membranpumpe ausgebildet ist.

5. Vorrichtung nach einem oder mehreren der Ansprüche 1 bis 4, **dadurch gekennzeichnet, dass** die Vorrichtung Einrichtungen zur periodischen Reinigung des Systems von Rohrleitungen, Pumpen und Ventilen aufweist.

6. Vorrichtung nach einem oder mehreren der Ansprüche 1 bis 4, **dadurch gekennzeichnet, dass** die Vorrichtung Mittel zur Identifizierung eines zu reinigenden und zu desinfizierenden Endoskops, Mittel zur Steuerung des Reinigungs- und Desinfektionszyklus in Abhängigkeit vom Endoskop-Typ und von bestimmten während des Zyklus gemessenen Parameter-Werten und Mittel zur Speicherung des Verlaufs des Reinigungs- und Desinfektionszyklus in einer Speichervorrichtung aufweist.

## Revendications

1. Dispositif de nettoyage et de désinfection d'endoscopes, comprenant au moins un bassin de lavage (19) dans lequel un endoscope (69) devant être nettoyé et désinfecté peut être placé, un boîtier (11), contenant entre autres un système de tuyaux, de pompes et de soupapes pour l'alimentation en liquides et en air à un endoscope placé dans un bassin de lavage, des moyens pour connecter les canaux d'un endoscope placé dans un bassin de lavage au système de tuyaux, de pompes et de soupapes, et des moyens de commande pour commander des pompes et des soupapes, le système de tuyaux, de pompes et de soupapes comprenant une unité de test à débit traversant (71) munie de canaux (72 à 78) et comportant une pluralité de sorties (79 à 85) connectant aux canaux, les sorties pouvant être connectées à un endoscope (69) placé dans un bassin de lavage (19), et une pluralité d'entrées (87 à 90) connectant aux canaux, les entrées étant connectées au reste du système des tuyaux, des pompes et des soupapes, tandis que chaque canal, menant à une sortie (80 à 85) qui peut être connectée à un canal d'endoscope, contient une soupape (39, 41, 42), **caractérisé en ce que** chaque canal menant à une sortie (80-85), qui peut être connectée à un canal d'endoscope, contient un capteur de pression (92 à 97) monté entre la soupape (39, 41, 42) et la sortie (80 à 85) du canal.

2. Dispositif selon la revendication 1, **caractérisé en ce que** chaque capteur de pression (92 à 97) se présente sous la forme d'un interrupteur de pression.

3. Dispositif selon la revendication 1, **caractérisé en ce qu'**une pompe d'augmentation de pression (98) est logée dans au moins l'un des canaux de l'unité de test à débit traversant, entre la soupape (39) et le capteur de pression (97).

4. Dispositif selon la revendication 3, **caractérisé en ce que** la pompe d'augmentation de pression (98) se présente sous la forme d'une pompe à diaphragme à entraînement pneumatique.

5. Dispositif selon une ou plusieurs des revendications 1 à 4, **caractérisé en ce que** le dispositif est muni de moyens, permettant de nettoyer périodiquement le système de tuyaux, de pompes et de soupapes.

6. Dispositif selon une ou plusieurs des revendications 1 à 4, **caractérisé en ce que** le dispositif est muni de moyens pour identifier un endoscope devant être nettoyé et désinfecté, de moyens pour commander le cycle de nettoyage et de désinfection selon le type d'endoscope et des valeurs de certains paramètres mesurées durant le cycle, et des moyens pour stocker dans une mémoire le déroulement du cycle de nettoyage et de désinfection.
